# EUROPÄISCHE PATENTSCHRIFT

(11) **EP 2 513 082 B1**
(45) Veröffentlichungstag und Bekanntmachung des Hinweises auf die Patenterteilung: **02.10.2013**
(21) Anmeldenummer: 10795292.1
(22) Anmeldetag: 10.12.2010
(51) Int. Cl.: C07D 317/30, C07D 319/12

(54) **NEUE DIOXOLAN- UND DIOXANDERIVATE UND EIN VERFAHREN ZUR DEREN HERSTELLUNG**
New dioxalane and dioxane derivatives and method for producing same
Nouveaux dérivés de dioxolane et de dioxane et leur procédé de fabrication

(30) Priorität: 15.12.2009 EP 09179298
(43) Veröffentlichungstag der Anmeldung: 24.10.2012
(73) Patentinhaber: Bayer CropScience AG, 40789 Monheim (DE)
(72) Erfinder: PAZENOK, Sergii, 42699 Solingen (DE); LUI, Norbert, 51519 Odenthal (DE); GERUS, Igor, 02094 Kiew (DE); BALABON, Olga, Kiew 02094 (UA)
(86) Internationale Anmeldenummer: PCT/EP2010/069380
(87) Internationale Veröffentlichungsnummer: WO 2011/073100

(56) Entgegenhaltungen:
- US-A- 2 556 135
- HIROYUKI FUKUDA, MASAHIRO HIROTA, YOSHIHIRO NAKASHIMA: "Spontaneous copolymerization of 4-methylene-1,3-dioxolanes with maleic anhydride", J. POLYM. SCI.: POLYMER CHEMISTRY EDITION, Bd. 20, 1982, Seiten 1401-1409, XP002578928,
- A. ROSSI, H. SCHINZ: HELVETICA CHIMICA ACTA, Bd. XXXII, Nr. VI, 1949, Seiten 1967-1974, XP002578929,
- MING-JIN FAN, GAO-QIANG LI, YONG-MIN LIANG: "DABCO catalyzed reaction of various nucleophiles with activated alkynes leading to the formation of alkenoic acid esters, 1,4-dioxane, morpholine and piperazinone derivatives", TETRAHEDRON, Bd. 62, 2006, Seiten 6782-6791, XP002578930,
- YAKAMBRAM PEDDURI, JOHN S. WILLIAMSON: "One-pot synthesis of highly substituted tetrahydrofurans from activated propargyl alcohols using Bu3P", TETRAHEDRON LETTERS, Bd. 49, 2008, Seiten 6009-6012, XP002578931,
- SALVATORE CABBIDU, CONSTANTINO FLORIS, STEFANA MELIS, FRANCESCA SOTGIU, GIOVANNI CERIONI: "Heterocyclic compounds studies . II. Synthesis of 1,4-benzodioxin, 1,4-benzoxathiin, 1,4-benzoxazine and 1,4-benzothiazine derivatives", J. HETEROCYCLIC CHEM., Bd. 23, 1986, Seiten 1815-1820, XP002578932,

## Beschreibung

Die vorliegende Erfindung betrifft neue Acylmethylen-1,3-dioxolane und Acylmethylen-1,4-dioxane, sowie ein neues Verfahren zur deren Herstellung.

Acylmethylen-1,3-dioxolane und Acylmethylen-1,4-dioxane sind wichtige Zwischenprodukte zur Herstellung von Pyrazolen und Anthranilsäureamiden, die als Insektizide Verwendung finden können.

In der Literatur ist bereits beschrieben, dass bestimmte Dioxolan-Derivate in Gegenwart von Säureanhydriden zu spontaner Polymerisation neigen *(*Spontaneous co-polymerization of 4-methylene-1,3-dioxolanes with maleic anhydride, Fukuda, Hiroyuki; Hirota, Masahiro; Nakashima, Yoshihiro, Nagoya Munic. Ind. Res. Inst., Rokuban, Japan, Journal of Polymer Science, Polymer Chemistry Edition (1982), 20(6), 1401-9.) Die erfindungsgemäßen Acylmethylen-1,3-dioxolane und Acylmethylen-1,4-dioxane sind jedoch nicht beschrieben; auch ein Verfahren zu deren Herstellung ist nicht in der Literatur beschrieben.

Aufgabe war es daher, ein Verfahren zur Verfügung zu stellen, welches es erlaubt, Acylmethylen-1,3-dioxolane und Acylmethylen-1,4-dioxane auf einfache Weise, ohne die im Stand der Technik beschriebenen Nachteile und in großtechnischem Maßstab aus bekannten 4-Methylene-1,3-dioxolanen und 6-Methylene-1,4-dioxanen herzustellen.

Erfindungsgemäß gelöst wurde die Aufgabe durch ein Verfahren zur Herstellung von neuen Acylmethylen-1,3-dioxolanen und Acylmethylen-1,4-dioxanen der Formel (I), 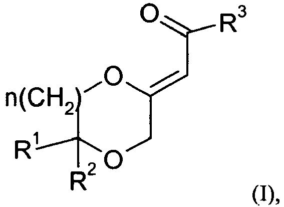 in welcher
- R¹ und R²: unabhängig voneinander für Wasserstoff, Alkyl, Halogenalkyl, Aryl oder Alkylaryl stehen,
- R¹, R²: weiterhin einen 4, 5, 6 oder 7-gliedrigen, gesättigten, gegebenenfalls substituierten Ring ausbilden können, welcher 1-2 Heteroatome aus der Reihe N,S,O enthalten kann,
- n: für 0 oder 1 steht,
- R³: für CX₃, (C=O)OAlkyl oder (C=O)OAryl steht,
- X: für Halogen steht,
indem man Verbindungen der Formel (II), 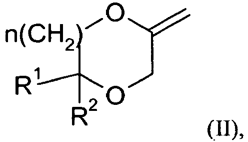 in welcher
R¹, R² und n die oben angegebenen Bedeutungen haben,
mit Verbindungen der allgemeinen Formel (III), beispielsweise Säurechloriden oder Anhydriden, 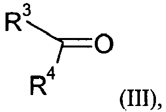 in welcher
R³ und X die oben angegebenen Bedeutungen haben und
R⁴ für X, O(C=O)R³ oder (CH₃)₃COO steht,
umsetzt zu erfindungsgemäßen Acylmethylen-1,3-dioxolanen und Acylmethylen-1 ,4-dioxanen der allgemeinen Formel (I).

Es ist als überraschend anzusehen, dass durch dass erfindungsgemäße Verfahren sich die neuen Acylmethylen-1,3-dioxolane und Acylmethylen-1,4-dioxane der Formel (I) selektiv und in hoher Ausbeute herstellen lassen, ohne dass störende Nebenreaktionen zu beobachten sind.

Beispiele für nach dem erfindungsgemäßen Verfahren herstellbare Verbindungen der Formel (I) sind:
1,1,1-Trichlor-3-(2,2-dimethyl-1,3-dioxolan-4-yliden)aceton, Methyl-3-(2,2-dimethyl-1,3-dioxolan-4-yliden)-2-oxopropanoat, Methyl -3-(1,3-dioxolan-4-yliden)-2-oxopropanoat, Ethyl-3-(2,2-dimethyl-1,3-dioxolan-4-yliden)-2-oxopropanoat, Ethyl-3-(5,5-dimethyl-1,4-dioxan-2-yliden)-2-oxopropanoat.

### Allgemeine Definitionen

Im Zusammenhang mit der vorliegenden Erfindung, umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen = (Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können.

Alkylaryl-Gruppen sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomere, wie auch die threo- und erythro-, und auch die optischen Isomere, beliebige Mischungen dieser Isomere, sowie die möglichen tautomeren Formen offenbart und beansprucht.

### 4-Methylen-1,3-dioxolan- und 6-Methylen-1,4-dioxan-Derivate der Formel (II)

Die bei der Durchführung des erfindungsgemäßen Verfahrens als Ausgangsstoffe verwendeten 4-Methylen-1,3-dioxolane and -6-Methylen-1,4-dioxane sind durch die Formel (II) allgemein definiert. 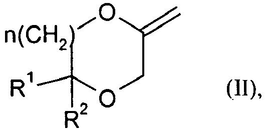 wobei
- R¹ und R²: unabhängig voneinander für Wasserstoff, Alkyl, Halogenalkyl, Aryl oder Alkylaryl stehen,
- R¹, R²: weiterhin einen 4, 5, 6 oder 7-gliedrigen, gesättigten, gegebenenfalls substituierten Ring ausbilden können, welcher 1-2 Heteroatome aus der Reihe N,S,O enthalten kann,
- R¹ und R²: unabhängig voneinander bevorzugt für Wasserstoff oder (C₁-C₁₂)-Alkyl stehen,
- R¹ und R²: unabhängig voneinander besonders bevorzugt für Wasserstoff oder Methyl stehen,
- n: für 0 oder 1 steht, bevorzugt und besonders bevorzugt für 0 steht.

Beispiele für erfindungsgemäß geeignete Ausgangsstoffe sind
2,2-Dimethyl-4-methylene-1,3-dioxolane, 4-methylene-1,3-dioxolane oder 2,2-dimethyl-6-methylene-1,4-dioxane. Die Verbindungen sind bekannt und können wie in
Gevorkyan, A. A.;et al. Khimiya Geterotsiklicheskikh Soedinenii (1991), (1), 33-6;
wie in J.Mattay et al Synthesis (1983), (3), 208-10;
oder wie in A.Kankaanpera et al., Acta Chemica Scandinavica (1966), 20(9), 2622 beschrieben, hergestellt werden.

### Verbindungen der Formel (III)

Die bei der Durchführung des erfindungsgemäßen Verfahrens als Ausgangsstoffe verwendeten Verbindungen sind durch die Formel (III) allgemein definiert: 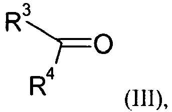 wobei
- R³: für CX₃, (C=O)OAlkyl oder (C=O)OAryl steht,
- R³: bevorzugt für CX₃, (C=O)OAlkyl steht,
- R³: besonders bevorzugt für (C=O)OAlkyl steht,
- X: für Halogen steht,
- X: bevorzugt und besonders bevorzugt für Chlor steht,
- R⁴: für X, O(C=O)R³ oder (CH₃)₃COO steht,
- R⁴: bevorzugt für X, besonders bevorzugt für Chlor steht.

Beispiele für im erfindungsgemäßen Verfahren einsetzbare Verbindungen der Formel (III) sind CF₃COCl, CCl₃COCl, ClCOCOOMe, ClCOCOOEt, (CF₃CO)₂O, CCl₃COF, CF₂HCOOCOC(CH₃)₃. Die Verbindungen sind kommerziell erhältlich.

### Reaktionsdurchführung.

Die Durchführung des erfindungsgemäßen Verfahrensschritts erfolgt vorzugsweise innerhalb eines Temperaturbereichs von -20°C bis 100°C, bevorzugt -10°C bis 40°C -, besonders bevorzugt bei Temperaturen von 0°C bis 30 °C. Der erfindungsgemäße Verfahrensschritt wird im Allgemeinen unter Normaldruck durchgeführt.

Die Reaktionszeit ist nicht kritisch und kann, in Abhängigkeit von der Ansatzgröße und Temperatur, in einem Bereich zwischen 30 min. und mehreren Stunden gewählt werden.

Bei der Durchführung des erfindungsgemäßen Verfahrensschritts setzt man 1 Mol des 4-Methylen-1,3-dioxolan- oder 6-Methylen-1,4-dioxan-Derivates der Formel (II) mit 0.8 Mol bis 1.5 Mol, vorzugsweise 0.9 Mol bis 1.2 Mol, besonders bevorzugt mit der äquimolaren Menge der Verbindung der Formel (III) um.

Die Acylierung wird in der Regel in Gegenwart einer Base durchgeführt. Geeignete Basen sind beispielsweise aliphatische, alicyclische, cyclische oder aromatische tertiäre Amine wie: Trimethylamin, Triethylamin, Tributylamin, Methyldiisopropylamin, Benzyldimethylamin, Pyridin, 2-, 3- oder 4-Methylpyridin, 2,3-Dimethylpyridin, 2-Methyl-5-ethylpyridin, 2,6-Dimethylpyridin, 2,5-Dimethylpyridin, 2,4,6-Trimethylpyridin, DBU, DABCO, N-Methylmorpholin, Dimethylcyclohexylamin. Anorganische Basen sind NaHCO₃, LiOH, NaOH, KOH, Na₂CO₃, K₂CO₃. Besonders bevorzugt verwendet man Triethylamin, Pyridin, Benzyldimethylamin, Dimethylpyridin, Pottasche.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt verwendet man Methylenchlorid, Dichlorethan, Toluol, Chlorbenzol, Methyltert.butylether oder THF.

Die Aufarbeitung des Reaktionsgemisches erfolgt wasserfrei durch Befreiung des Gemisches von Salzen (Filtration) und Entfernung der Lösemittel im Vakuum. Wässrige Aufarbeitung ist auch möglich. Es ist auch möglich, das Gemisch ohne vorherige Isolierung weiter umzusetzen.

Die Reinheit der Verbindungen der Formel (I) ist sehr hoch und liegt in Bereich von 95-97 %, welche ohne Reinigungsschritt weiter eingesetzt werden können. Insbesondere zeichnet sich die erfindungsgemäße Umsetzung durch den Einsatz günstiger Rohstoffe, sowie durch auch im großtechnischen Maßstab besonders gut und einfach durchzuführende Prozeßführung aus.

Die erfindungsgemäßen Verbindungen der Formel (I) sind wertvolle Zwischenprodukte in der Synthese von Pyrazolen, die wichtige Bausteine für die Herstellung von insektizid wirksamen Anthranilsäureamiden (WO2007/112893, WO2007/144100 darstellen und können z.B. gemäß dem nachfolgenden Schema (I) weiter umgesetzt werden. Beispielsweise lassen sich die folgenden Verbindungen durch die weitere Umsetzung der Verbindungen der Formel (I) gemäß Schema (I) erhalten:

Methyl 1-(3-chloropyridin-2-yl)-5-hydroxy-3-(hydroxymethyl)-4,5-dihydro-1H-pyrazole-5-carboxylate (vgl. Herstellbeispiel Nr. 6); Ethyl 1-(3-chloropyridin-2-yl)-5-hydroxy-3-(hydroxymethyl)-4,5-dihydro-1H-pyrazole-5-carboxylate (Herstellbeispiel Nr. 7); Methyl 1-(3-chloropyridin-2-yl)-3-{[(methylsulfonyl)oxy]methyl}-1H-pyrazole-5-carboxylate (Herstellbeispiel Nr. 8); Methyl 3-(chloromethyl)-1-(3-chloropyridin-2-yl)-1H-pyrazole-5-carboxylate (Herstellbeispiel Nr. 9). 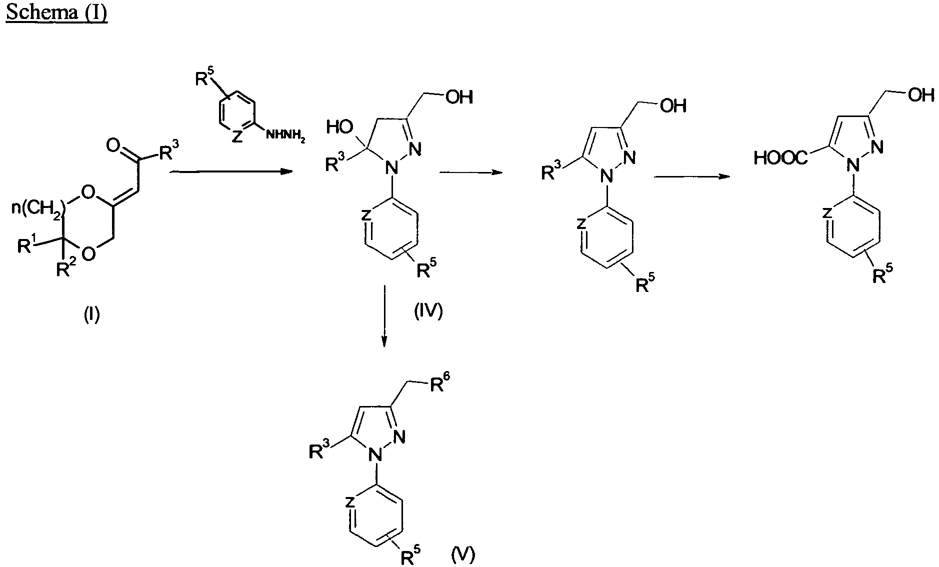 wobei
- R¹, R², R³: und n die oben angegebenen Bedeutungen haben,
- R⁵: für Halogen, Cyano, Nitro, Alkyl, Cycloalkyl, Halogenalkyl, Halogencycloalkyl, Alkoxy, Halogenalkoxy, Alkylamino, Dialkylamino, Cycloalkylamino steht,
- R⁶: für Halogen, OSO₂Me, O(C=O)CH₃ steht,
- Z: für CH, N steht.

### Herstellbeispiele

Die folgenden Herstellbeispiele illustrieren die Erfindung, ohne sie zu beschränken. Insbesondere zeigen die Herstellbeispiele 1A, 1 bis 4 und 5 die Herstellung von Verbindungen der Formel (I) nach dem erfindungsgemäßen Verfahren, die Herstellbeispiele 6 bis 9 und Beispiel 10 belegen die Verwendung der Verbindungen der Formel (I) als Zwischenprodukte für die Weiterreaktion zu Pyrazolen, welche wichtige Bausteine in der Synthese von insektizid wirksamen Anthranilsäureamiden darstellen.

### Beispiel 1A

### Methyl (3E)-3-(2,2-dimethyl-1,3-dioxolan-4-ylidene)-2-oxopropanote.

Zu der Lösung von 2,2-Dimethyl-4-Methylene-1,3-dioxolan (114. g, 1 mol) und Pyridin (79 g, 1 mol) in 200 ml CH₂Cl₂ wurde die Lösung von Methyloxalylchlorid (122 g, 1 mol) in 100 ml CH₂Cl₂ bei 0°C zugegeben. Das Reaktionsgemisch wurde 1 Std. bei 20°C nachgerührt und mit 500 mL Wasser verdünnt. Die organische Phase wurde abgetrennt, mit MgSO₄ getrocknet und CH₂Cl₂ in Vakuum entfernt. Ausbeute 180 g (90 %).
Smp. 61-63 °C.
¹H NMR (CDCl₃) δ: 6.88 (1H, s), 5.10 (2H, s), 3,8 (3H,s), 1,5 (6H, s);

### Beispiel 1B

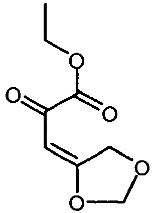

### (E)-Ethyl 3-(1,3-dioxolan-4-ylidene)-2-oxopropanoate

Die Lösung von Ethyl oxalylchloride (10.3 g, 75.5 mmol) in CH₂Cl₂ (10 mL) wurde zu der Lösung von Methylendioxolan (6.5 g, 75.5 mmol) und Pyridine (6.3 g, 80 mmol) in CH₂Cl₂) (80 mL) bei 0 °C zugegeben. Das Gemisch wurde 10 Std. bei 5 °C gerührt und auf 200 mL Wasser gegossen. Das Produkt wurde mit 200 ml Hexan extrahiert. Die Hexan-Lösung wurde über MgSO₄ getrocknet und eingeengt. Man erhielt 11,9 g (85 %) des Produktes mit einem Sdp. 106-110 °C / 0.5 mmHg.
¹H NMR (CDCl₃) δ: 6.60 (1H, s, C*H*), 5.45 (2H, s, C*H*₂O), 5.01 (2H, s, C*H*₂O)*,* 4.33 (2H, q, OC*H*₂), 1.38 (3H, t, C*H*₃).

### Beispiel 1

### (E)-3-(1,3-Dioxolan-4-yliden)-1,1,1-trifluoropropan-2-on

Zur Lösung von 4-Methylene-1,3-dioxolan (16.1 g, 187 mmol) und Pyridin (16.3 g, 15.3 mL, 205 mmol) in 150 ml CH₂Cl₂ wurde die Lösung von Trifluoressiganhydrid (39.3 g, 187 mmol) in 30 ml CH₂Cl₂ bei 0°C zugetropft. Das Reaktionsgemisch wurde 20 Stunden bei 5 °C gerührt und mit 100 mL Wasser verdünnt. Das Produkt wurde mit Toluol extrahiert, die organische Phase mit MgSO₄ getrocknet und Toluol im Vakuum entfernt. Das Produkt wurde durch Destillation gereinigt. Ausbeute von (*E*)-3-(1,3-Dioxolan-4-yliden)-1,1,1-trifluoropropan-2-on ist 28.3 g (83%), Sdp.: 83-85 °C / 15 mbar.

### Analytische Charakterisierung

¹H NMR (CDCl₃) δ: 6.16 (1H, s), 5.51 (2H, s), 5.05 (2H, s) ppm;
¹³C NMR (125 MHz, CDCl₃) δ: 180.20 (q), 175.76, 116.29 (q), 99.15, 90.83, 71.02 ppm;
¹⁹F NMR (CDCl₃) δ: -77.55 (s) ppm.

### Beispiel 2

### (E)-3-(2,2-Dimethyl-1,3-dioxolan-4-yliden)-1,1,1-trifluoropropan-2-on

Die Durchführung erfolgt wie in Beispiel 1 beschrieben, jedoch unter Verwendung von 2,2-Dimethyl-4-methylen-1,3-dioxolan anstelle von 4-Methylen-1,3-dioxolan.

Ausbeute (80%), Sdp. 72-74 °C / 20 mbar.

### Analytische Charakterisierung

¹H NMR (CDCl₃) δ: 6.03 (1H, s), 5.12 (2H, s), 1.59 (6H, s); ¹³C NMR (CDCl₃) δ: 180.09 (q), 176.76, 117.08, 116.39 (q), 90.01, 71.19, 24.86 ppm;
¹⁹F NMR (CDCl₃) δ: -77.63 (s) ppm.

### Beispiel 3

### (E)-1,1,1-Trichlor-3-(1,3-dioxolan-4-yliden)propan-2-on

Zur Lösung von 4-Methylen-1,3-dioxolan (4.7 g, 55 mmol) und Pyridin (4.8 g, 4.5 mL, 60 mmol) wurde die Lösung von Trichloracetylchlorid (10.0 g, 55 mmol) in 20 ml CH₂Cl₂ bei 0°C zugegeben. Das Reaktionsgemisch wurde 4 Stunden bei 20°C nachgerührt und mit 100 mL Wasser verdünnt. Das Produkt wurde mit Hexan extrahiert, die organische Phase mit MgSO₄ getrocknet und Hexan in Vakuum entfernt. Das Produkt wurde durch Kristallisation aus Hexan gereinigt.

Ausbeute von (*E*)-1,1,1-Trichlor-3-(1,3-dioxolan-4-yliden)propan-2-on 7.5 g (59%),
Smp. 64 °C.

### Analytische Charakterisierung

¹H NMR (CDCl₃) δ: 6.38 (1H, br. s), 5.46 (2H, s), 5.02 (2H, s) ppm;
¹³C NMR (CDCl₃) δ: 181.45, 174.71, 98.92, 96.50, 89.87, 70.55 ppm

### Beispiel 4

### (E)-1,1,1-Trichlor-3-(2,2-dimethyl-1,3-dioxolan-4-yliden)propan-2-on

Man arbeitet wie in Beispiel 3 beschrieben, verwendet jedoch 2,2-Dimethyl-4-methylen-1,3-dioxolan anstelle von 4-Methylen-1,3-dioxolan.

Ausbeute von 1,1,1-Trichlor-3-(2,2-dimethyl-1,3-dioxolan-4-yliden)propan-2-on:
(72%), Sdp. 140 °C / 0.5 mbar., Smp. 40-42°C.

### Analytische Charakterisierung:

¹H NMR (CDCl₃) δ: 6.28 (1H, br. t), 5.12 (2H, br. d), 1.59 (6H, s) ppm;
¹³C NMR (CDCl₃) δ: 181.25, 175.24, 116.15, 96.16, 88.97, 70.60, 25.05 ppm

### Beispiel 5

### Ethyl (3E)-3-(2,2-dimethyl-1,3-dioxolan-4-yliden)-2-oxopropanoat

Man arbeitet wie in Beispiel 4 beschrieben, verwendet jedoch Ethylchloro(oxo)acetat anstelle von Trichloracetylchlorid. Ausbeute 85 %, Öl.

### Analytische Charakterisierung:

¹H NMR (CDCl₃) δ: 6.28 (1H, br. t), 5.12 (2H, br. d), 1.59 (6H, s) ppm;
¹³C NMR (CDCl₃) δ: 181.25, 175.24, 116.15, 96.16, 88.97, 70.60, 25.05 ppm.

### Beispiel 6

### Methyl 1-(3-chloropyridin-2-yl)-5-hydroxy-3-(hydroxymethyl)-4,5-dihydro-1H-pyrazol-5-carboxylat.

Das Gemisch von Methyl (3E)-3-(2,2-dimethyl-1,3-dioxolan-4-yliden)-2-oxopropanoate (20 g., 0.1 mol) und 2-Hydrazino-3-chlorpyridin (21.4 g, 0.15 mol) in 150 ml Isopropanol wurde 8 bei RT gerührt. Der Niederschlag wurde abfiltriert und mit Hexan gewaschen. Man erhielt 27,6 g (85 %) des Produktes als hell-gelben Feststoff mit einem Smp. von 113-115 °C.

¹H NMR (DMSO d₆) δ: 7.99 (1H, d); 7,65 (1H, d); 6.85 (1H, dd); 6.4 (1H, bs); 4,51 (2H, br. s); 3,25 (1H, d); 3,05 (1H,d), 2,55 (s, 1 H) ppm.

### Beispiel 7

### Ethyl 1-(3-chloropyridin-2-yl)-5-hydroxy-3-(hydroxymethyl)-4,5-dihydro-1H-pyrazol-5-carboxylat

Das Gemisch von Ethyl (3E)-3-(2,2-dimethyl-1,3-dioxolan-4-yliden)-2-oxopropanoat (21.4 g., 0.1 mol) und 2-Hydrazino-3-chlorpyridin (21.4 g , 0.15 mol) in 150 ml Ethanol wurde 8 bei RT gerührt. Ethanol wurde in Vakuum entfernt und der Rückstand in 200 ml Methyltert.butylether aufgenommen. Die organische Phase wurde 3 mal mit jeweils 50 ml von 1 % HCl gewaschen und eingeengt. Man erhielt 36 g (86 % Ausbeute) des Produktes als zähes Öl mit der Reinheit (HPLC) von 97 %.

### Analytische Charakterisierung

¹H NMR (DMSO d₆) δ: 7.99 (1H, d); 7,65 (1h, d); 6.85 (1H, dd); 6.0 (OH, bs); 4,51 (2H, br. s); 4.25 (2H, q); 3,25 (1H, d); 3,05 (1H,d), 1,28 (t, 3H) ppm

### Beispiel 8

### Methyl 1-(3-chloropyridin-2-yl)-3-{[(methylsulfonyl)oxy]methyl}-1H-pyrazol-5-carboxylat

Methyl 1-(3-chloropyridin-2-yl)-5-hydroxy-3-(hydroxymethyl)-4,5-dihydro-1H-pyrazol-5-carboxylat (28.5 g, 0.1 mol) und 15 g Triethylamin wurden in 150 ml THF vorgelegt und die Lösung auf 5°C gekühlt. 11,4 g Mesylenchlorid wurde bei 0-5°C innerhalb 20 min zugegeben und das Gemisch bei 0°C 2 nachgerührt. Das Reaktionsgemisch wurde mit Wasser verdünnt und das Produkt mit Ethylacetate extrahiert. Die Lösung wurde gewaschen getrocknet und eingeengt. Der zähe ölige Rückstand 31 g enthielt nach LC/MS 98 % des Produktes mit m/z 345.

### Beispiel 9

### Methyl 3-(chloromethyl)-1-(3-chloropyridin-2-yl)-1H-pyrazol-5-carboxylat

Methyl 1-(3-chloropyridin-2-yl)-5-hydroxy-3-(hydroxymethyl)-4,5-dihydro-1H-pyrazol-5-carboxylat (28.5 g, 0.1 mol) wurde in 100 ml CH₂Cl₂ gelöst und die Lösung auf 5°C gekühlt. SOCl₂ (0.12 mol) in 30 ml CH₂Cl₂ wurde langsam bei dieser Temperatur zugetropft. Das Gemisch wurde 4 bei RT nachgerührt und in Vakuum eingeengt. Das Produkt wurde durch Säulenchromatographie auf SiO₂ gereinigt (Eluent Hexan/Etylacetat). Öl.

### Analytische Charakterisierung

¹H NMR (CD₃CN) δ: 8,52 (1H, d); 8,06 (1H,d), 7,55 (1H, dd); 7,10 (1H, s); 4,75 (2H,s); 3,75 (3H,s) ppm.

### Beispiel 10

### Methyl 1-(3-chloropyridin-2-yl)-3-(hydroxymethyl)-1H-pyrazole-5-carboaxylat

Zu der Suspension von 28,5 g (0,1 mol) Methyl 1-(3-chloropyridin-2-yl)-5-hydroxy-3-(hydroxymethyl)-4,5-dihydro-1H-pyrazole-5-carboxylat in 100 ml Methanol wurde HCl-Lösung (9,1 g, als 4 % Lösung in Methanol) zugegeben. Nach ca. 30 Min bei 25-30°C war eine klare, gelbe Lösung entstanden. Methanol wurde im Vakuum entfernt und der Niederschlag mit Wasser gewaschen. Ausbeute 26,7 g., 100 %. Schmp. 104°C.

### Analytische Charakterisierung

¹H NMR (DMSO d₆) δ: 8,52 (1H,d); 8,06 (1H,d), 7,55 (1H, dd); 7,10 (1H, s); 5,4 (1H, b.s) 4,5 (2H,s); 3,75 (3H,s) ppm.

## Patentansprüche

1. Acylmethylen-1,3-dioxolane und Acylmethylen-1,4-dioxane der allgemeinen Formel (I) 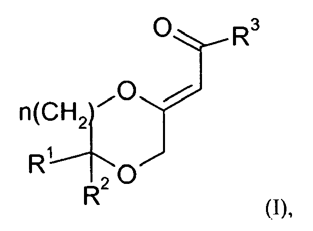 in welcher
R¹ und R² unabhängig voneinander für Wasserstoff, Alkyl, Halogenalkyl, Aryl oder Alkylaryl stehen,
R¹, R² weiterhin einen 4, 5, 6 oder 7-gliedrigen, gesättigten, gegebenenfalls substituierten Ring ausbilden können, welcher 1-2 Heteroatome aus der Reihe N,S,O enthalten kann,
n für 0 oder 1 steht,
R³ für CX₃, (C=O)OAlkyl oder (C=O)OAryl steht,
X für Halogen steht.

2. Verbindungen der Formel (I) gemäß Anspruch 1, **dadurch gekennzeichnet, dass**
R¹ und R² unabhängig voneinander für Wasserstoff oder Methyl stehen,
n für 0 steht,
R³ für CX₃ oder (C=O)OAlkyl steht,
X für Chlor steht.

3. Verbindungen der Formel (I) gemäß einem der Ansprüche 1 oder 2, **dadurch gekennzeichnet, dass**
R¹ und R² unabhängig voneinander für Wasserstoff oder Methyl stehen,
n für 0 steht,
R³ für (C=O)OMethyl steht,

4. Verfahren zur Herstellung von Verbindungen der allgemeinen Formel (I) gemäß einem der Ansprüche 1,2 oder 3, **dadurch gekennzeichnet, dass** man Verbindungen der Formel (11), 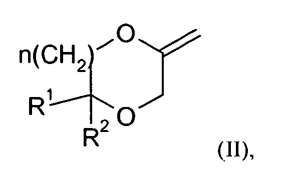 in welcher
R¹, R² und n die Bedeutung gemäß einem der Ansprüche 1, 2 oder 3 haben,
mit Verbindungen der allgemeinen Formel (III), 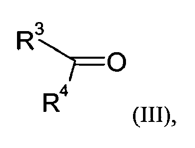 in welcher
R³ die Bedeutung gemäß einem der Ansprüche 1, 2 oder 3 hat,
X die Bedeutung gemäß einem der Ansprüche 1 oder 2 hat und
R⁴ für X, O(C=O)R³ oder (CH₃)₃COO steht,
umsetzt zu Verbindungen der allgemeinen Formel (I).

5. Verfahren zur Herstellung von Verbindungen der Formel (I) gemäß Anspruch 4, **dadurch gekennzeichnet, dass** R¹, R², R³, n und X die Bedeutung gemäß einem der Ansprüche 1, 2 oder 3 haben und R⁴ für X steht.

6. Verfahren zur Herstellung von Verbindungen der Formel (I) gemäß einem der Ansprüche 4 oder 5, **dadurch gekennzeichnet, dass** das Verfahren in einem Temperaturbereich von -20°C bis 100°C durchgeführt wird.

7. Verfahren zur Herstellung von Verbindungen der Formel (I) gemäß einem der Ansprüche 4 bis 6, **dadurch gekennzeichnet, dass** 1 Mol einer Verbindung der Formel (II) mit 0.8 bis 1.5 Mol einer Verbindung der Formel (III) umgesetzt wird.

8. Verwendung von Verbindungen der allgemeinen Formel (I) zur Herstellung von Pyrazolen und Anthranilsäureamiden.

## Claims

1. Acylmethylene-1,3-dioxolanes and acylmethylene-1,4-dioxanes of the general formula (I) 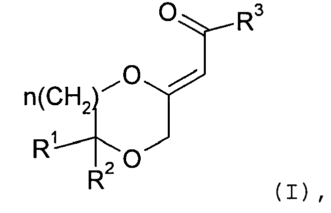 in which
R¹ and R², independently of one another, are hydrogen, alkyl, haloalkyl, aryl or alkylaryl,
R¹, R² futhermore can form a 4-, 5-, 6- or 7-membered, saturated, optionally substituted ring which can contain 1-2 heteroatoms from the series N, S, O,
n is 0 or 1,
R³ is CX₃, (C=O) Oalkyl or (C=O)Oaryl,
X is halogen.

2. Compounds of the formula (I) according to Claim 1, **characterized in that**
R¹ and R², independently of one another, are hydrogen or methyl,
n is 0,
R³ is CX₃ or (C=O) Oalkyl,
X is chlorine.

3. Compounds of the formula (I) according to one of Claims 1 or 2, **characterized in that**
R¹ and R², independently of one another, are hydrogen or methyl,
n is 0,
R³ is (C=O)Omethyl,

4. Process for the preparation of compounds of the general formula (I) according to one of Claims 1, 2 or 3, **characterized in that** compounds of the formula (II), 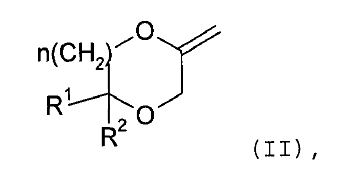 in which
R¹, R² and n have the meaning according to one of Claims 1, 2 or 3,
are reacted with compounds of the general formula (III), 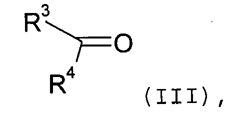 in which
R³ has the meaning according to one of Claims 1, 2 or 3,
X has the meaning according to one of Claims 1 or 2 and
R⁴ is X, O(C=O)R³ or (CH₃)₃COO,
to give compounds of the general formula (I).

5. Process for the preparation of compounds of the formula (I) according to Claim 4, **characterized in that** R¹, R², R³, n and X have the meaning according to one of Claims 1, 2 or 3 and R⁴ is X.

6. Process for the preparation of compounds of the formula (I) according to one of Claims 4 or 5, **characterized in that** the process is carried out in a temperature range from -20°C to 100°C.

7. Process for the preparation of compounds of the formula (I) according to one of Claims 4 to 6, **characterized in that** 1 mol of a compound of the formula (II) is reacted with 0.8 to 1.5 mol of a compound of the formula (III).

8. Use of compounds of the general formula (I) for the preparation of pyrazoles and anthranilamides.

## Revendications

1. Acylméthylène-1,3-dioxolanes et acylméthylène-1,4-dioxanes de formule générale (I) 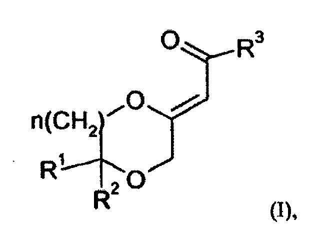 dans laquelle
R¹ et R² représentent indépendamment l'un de l'autre hydrogène, alkyle, halogénoalkyle, aryle ou alkylaryle, R¹, R² peuvent également former un cycle à 4, 5, 6 ou 7 éléments, saturé, éventuellement substitué, qui peut contenir 1 à 2 hétéroatomes de la série N, S, O,
n représente 0 ou 1,
R³ représente CX₃, (C=O)O-alkyle ou (C=O)-O-aryle,
X représente halogène.

2. Composés de formule (I) selon la revendication 1, **caractérisés en ce que**
R¹ et R² représentent indépendamment l'un de l'autre hydrogène ou méthyle,
n représente 0,
R³ représente CX₃ ou (C=O)O-alkyle,
X représente chlore.

3. Composés de formule (I) selon l'une quelconque des revendications 1 ou 2, **caractérisés en ce que**
R¹ et R² représentent indépendamment l'un de l'autre hydrogène ou méthyle,
n représente 0,
R³ représente (C=O)O-méthyle.

4. Procédé de fabrication de composés de formule générale (I) selon l'une quelconque des revendications 1, 2 ou 3, **caractérisé en ce que** des composés de formule (II) 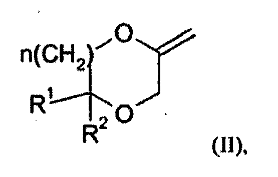 dans laquelle
R¹, R² et n ont la signification selon l'une quelconque des revendications 1, 2 ou 3,
sont mis en réaction avec des composés de formule générale (III) 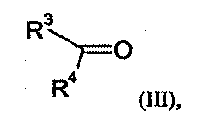 dans laquelle
R³ a la signification selon l'une quelconque des revendications 1, 2 ou 3,
X a la signification selon l'une quelconque des revendications 1 ou 2 et
R⁴ représente X, O(C=O)R³ ou (CH₃)₃COO,
pour former des composés de formule générale (I).

5. Procédé de fabrication de composés de formule (I) selon la revendication 4, **caractérisé en ce que** R¹, R², R³, n et X ont la signification selon l'une quelconque des revendications 1, 2 ou 3 et R⁴ représente X.

6. Procédé de fabrication de composés de formule (I) selon l'une quelconque des revendications 4 ou 5, **caractérisé en ce que** le procédé est réalisé dans une plage de température allant de -20 °C à 100 °C.

7. Procédé de fabrication de composés de formule (I) selon l'une quelconque des revendications 4 à 6, **caractérisé en ce que** 1 mol d'un composé de formule (II) est mis en réaction avec 0,8 à 1,5 mol d'un composé de formule (III).

8. Utilisation de composés de formule générale (I) pour la fabrication de pyrazoles et d'amides de l'acide anthranilique.
